# EUROPEAN PATENT SPECIFICATION

(11) **EP 2 446 914 B1**
(45) Date of publication and mention of the grant of the patent: **06.08.2014**
(21) Application number: 10791964.9
(22) Date of filing: 08.06.2010
(51) Int. Cl.: A61M 21/02, A47C 3/02, A61H 7/00, A61H 15/00

(54) **RELAXATION DEVICE**
ENTSPANNUNGSVORRICHTUNG
DISPOSITIF DE RELAXATION

(30) Priority: 25.06.2009 JP 2009150773
(43) Date of publication of application: 02.05.2012
(73) Proprietor: Panasonic Corporation, Osaka 571-8501 (JP)
(72) Inventor: MORIKAWA, Daisuke, Osaka 540-6207 (JP); MICHIMORI, Akihiro, Osaka 540-6207 (JP)
(74) Representative: Appelt, Christian W.
(86) International application number: PCT/JP2010/059655
(87) International publication number: WO 2010/150646

(56) References cited:
- EP-A1- 1 974 709
- JP-A- 11 137 626
- JP-A- 2000 042 113
- JP-A- 2000 300 374
- JP-A- 2001 149 164
- JP-A- 2001 178 576
- JP-A- 2001 190 360
- JP-A- 2003 250 851
- JP-A- 2004 073 550
- JP-A- 2007 313 125
- JP-A- 2009 112 790
- JP-B2- 3 911 944
- JP-B2- 4 274 019
- US-A1- 2007 273 187
- US-A1- 2008 030 053
- US-A1- 2008 214 972

## Description

### TECHNICAL FIELD

The present invention relates to a relaxation device that has a relaxation effect on a user.

### BACKGROUND ART

A relaxation device known in the prior art produces a relaxation effect by rocking the body of a user who sits on a chair-type body support (refer to patent document 1).

The relaxation device produces a rocking motion at a frequency of 1 Hz or less. At the same time, the relaxation device gradually changes and varies the rocking motion in varying widths of 0.2 Hz to improve the relaxation effect.

Document US 2007/0273187 A1 discloses a massage chair. From document US 2008/ 0214972 A1, a therapeutic device for inducing blood pressure modulation is known. Documents US 2008/0030053 A1 and EP 1 974 709 A1 disclose a massage machine of chair type and a chair-type massage machine, respectively. From document JP 2007 300374 A, a relaxing device is known.

Furthermore, documents JP 2000 042113 A, JP 11 137626 A, JP 2001 178576, A, JP 2001 149164 A, JP 2001 190360 A and JP 3911944 B2 disclose respective relaxing devices and relax inducing apparatus. From documents JP 2007 313125 A, JP 2003 250851 A, JP 2009 112790 A, JP 4274019 B2 and JP 2004 073550 A, massage machines and a body operating device are known, respectively.

### PRIOR ART DOCUMENT

### PATENT DOCUMENT

Patent Document 1: Japanese Laid-Open Patent Publication No. 2000-42112

### SUMMARY OF THE INVENTION

### PROBLEMS THAT ARE TO BE SOLVED BY THE INVENTION

The relaxation device of the prior art has a relaxation effect on a user. However, it is difficult for the user to enjoy the rocking and the user may become bored.

It is an object of the present invention to provide a relaxation device that has a relaxation effect on a user and produces a floating feel for the user so that the user can enjoy rocking.

### MEANS FOR SOLVING THE PROBLEMS

The invention refers to the relaxation device of claim 1. Preferred embodiments are disclosed in the dependent claims.

To solve the above problem, one aspect of the present invention provides a relaxation device including a body support unit that supports a user's body, a rocking drive unit that rocks the body support unit toward the front and rear along an arc-shaped movement path, and a control unit that controls the rocking drive unit to perform relaxation rocking with the body support unit. The control unit controls the rocking drive unit so that the rocking of the body support unit is stopped for a predetermined time when a rocking direction of the body support unit changes. The control unit controls a first predetermined time, for when the rocking direction of the body support unit changes from a forward direction to a rearward direction, to be different from a second predetermined time, for when the rocking direction of the body support unit changes from the rearward direction to the forward direction.

It is preferable that the control unit control the rocking drive unit so that the first predetermined time becomes longer than the second predetermined time.

It is preferable that the control unit gradually increase at least either one of the first predetermined time and the second predetermined time as the relaxation rocking is repeated.

It is preferable that the control unit increase and decrease at least either one of the first predetermined time and the second predetermined time in an irregular manner as the relaxation rocking is repeated.

It is preferable that the relaxation device further include a lifting mechanism arranged in the body support unit to lift the user. The control unit controls the lifting mechanism so that the user is lifted in at least either one of the first predetermined time and the second predetermined time.

It is preferable that the body support unit include a seat on which a user can sit and a backrest fixed in a reclinable manner to the rear of the seat, and the control unit recline the backrest to set the backrest in a reclining state during at least either one of the first predetermined time and the second predetermined time.

### EFFECT OF THE INVENTION

The present invention provides a relaxation device that has a relaxation effect on a user and produces a floating feel for the user so that the user can enjoy rocking.

### BRIEF DESCRIPTION OF THE DRAWINGS

Fig. 1 is a schematic diagram showing a relaxation device in a first embodiment;
Fig. 2(a) is a block diagram showing a controller, a reclining mechanism, and an air pump, and Fig. 2(b) is a perspective view showing a body support;
Fig. 3 is a time chart showing the rocking position of the body support;
Fig. 4(a) is a time chart showing the rocking position of the body support, Fig. 4(b) is a chart illustrating a first predetermined time that increases as the number of rocking reciprocations of the relaxation device increases; and
Fig. 5 is a chart illustrating the first predetermined time that varies as the number of rocking reciprocations of the relaxation device increases.

### EMBODIMENTS OF THE INVENTION

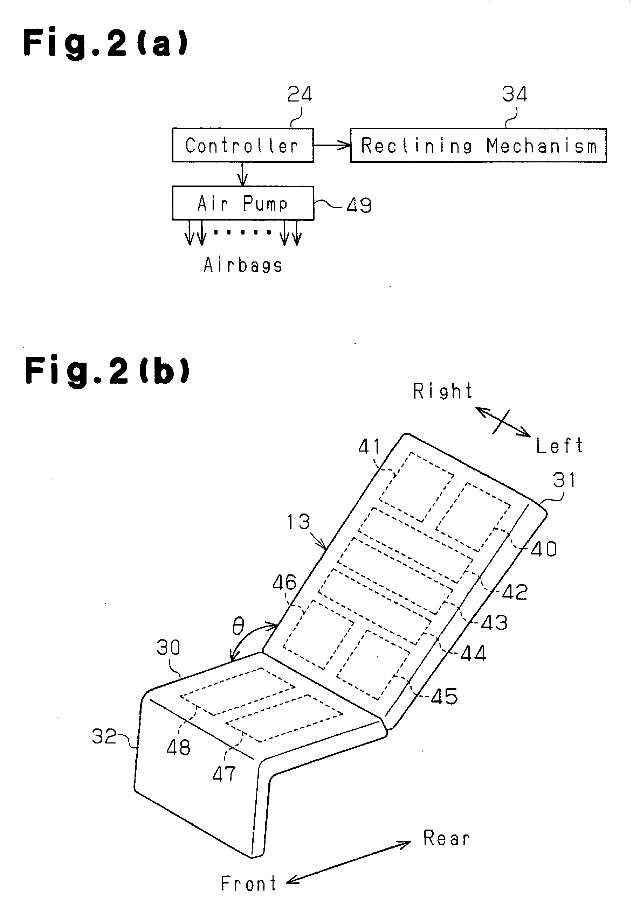

A relaxation device according to a first embodiment of the present invention will now be described with reference to the drawings. In this specification, right and left refers to the right and left in a state in which a user is sitting on the relaxation device (refer to Fig. 2(b)).

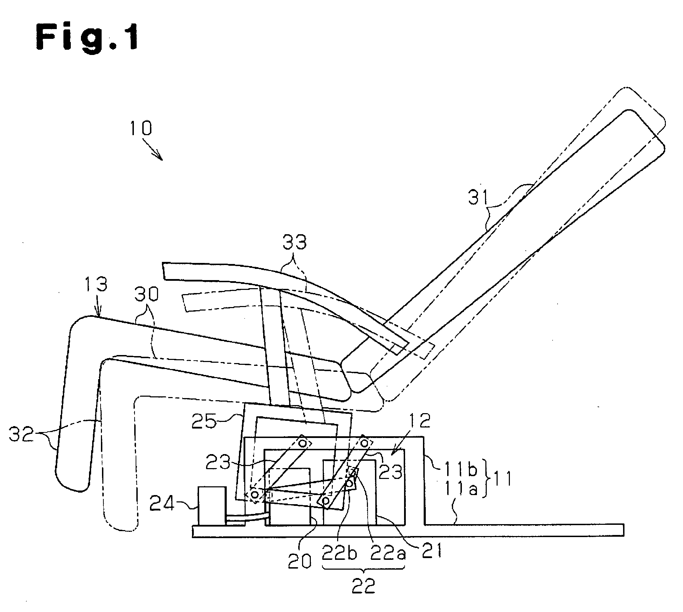

Fig. 1 is a schematic diagram showing a relaxation device 10 of the first embodiment. As shown in Fig. 1, the relaxation device 10 includes a pedestal 11 of which bottom portion 11a is placed on a floor surface. A rocking driver 12, which serves as a rocking drive unit, is arranged on the pedestal 11. A body support 13, which serves as a body support unit, is rocked by the rocking driver 12.

The rocking driver 12 includes a motor 20, a reduction gear 21, a crank mechanism 22, and a link 23.

The motor 20 is arranged on an upper surface of the bottom portion 11 a. A controller 24, which serves as a control unit, is arranged on the upper surface of the bottom portion 11a. The controller 24 controls the driving of the motor 20. The reduction gear 21 is arranged on the upper surface of the bottom portion 11a of the pedestal 11 and drive-coupled to the motor 20 to decelerate and output the driving force of the motor 20.

The crank mechanism 22 is formed by two connection members 22a and 22b to convert the rotational driving motion of the reduction gear 21 into an arc motion having an extremely large radius. The connection member 22a includes a basal end connected to an output shaft of the reduction gear 21 in an integrally rotatable manner. Further, the connection member 22a includes a distal end connected to a basal end of the connection member 22b in a rotatable manner. The connection member 22b includes a distal end connected to a lower portion of a tetragonal frame-shaped base 25. The body support 13 is fixed to the tetragonal frame-shaped base 25.

The pedestal 11 includes the bottom portion 11a and a support frame 11b, which projects in an upward direction from the bottom portion 11a. Two links 23 each have a basal end coupled in a rotatable manner to and spaced apart by a predetermined distance from an upper portion of the support frame 11 b. Each link 23 has a distal end coupled in a rotatable manner to a lower portion of the base 25. Thus, the two links 23 pivot about their basal ends. The driving force from the crank mechanism 22 rocks the body support 13, which is fixed to the base 25, toward the front and rear along an arc-shaped movement path.

The chair-shaped body support 13 is fixed to an upper portion of the base 25. The body support 13 includes a seat 30, a backrest 31, an ottoman 32, and armrests 33. The seat 30 is fixed to the base 25 in an integrally movable manner. The backrest 31, which is reclinable, is fixed to the rear of the seat 30. The ottoman 32 is fixed to the front of the seat 30. The armrests 33 are arranged on the right side and left side of the seat 30.

As shown in Fig. 2, the controller 24 controls a reclining mechanism 34, which reclines the backrest 31, to recline the backrest 31 when necessary.

Two shoulder airbags 40 and 41 are arranged in the upper left and right parts of the backrest 31 in correspondence with the left and right shoulders of a user. Shoulder airbags 42, 43, and 44 are arranged in the middle part of the backrest 31. Hip airbags 45 and 46 are arranged in the lower left and right sides of the backrest 31 in correspondence with the left and right hips of a user. Two seat airbags 47 and 48 are arranged in the seat 30 in correspondence with the buttock and thighs of a user. The airbags 40 to 48 are connected by a connection hose (not shown) to an air pump 49, which is arranged in a lower portion of the seat 30. The controller 24 controls the driving of the air pump 49 to inflate and deflate the airbags 40 to 48. The airbags 40 to 48 and the air pump 49 form a lifting mechanism.

The operation of the relaxation device 10 will now be described with reference to Figs. 1 to 3. The controller 24 controls the motor 20 to rock the body support 13 toward the front and rear along an arc-shaped movement path. The controller 24 controls the motor 20 so that the rocking of the body support 13 stops for a predetermined time when the rocking direction of the body support 13 changes. In the illustrated example, the controller 24 executes control so that a first predetermined time for when the rocking direction of the body support 13 changes from the forward direction to the rearward direction differs from a second predetermined time for when the rocking direction of the body support 13 changes from the rearward direction to the forward direction. As a result, the rocking has a relaxation effect on the user. Further, the user experiences a floating feel during the first predetermined time and the second predetermined time and thus can enjoy the rocking. This allows the user to experience a variety of floating feels. The first and second predetermined times are also referred to as a front position still period and a rear position still period.

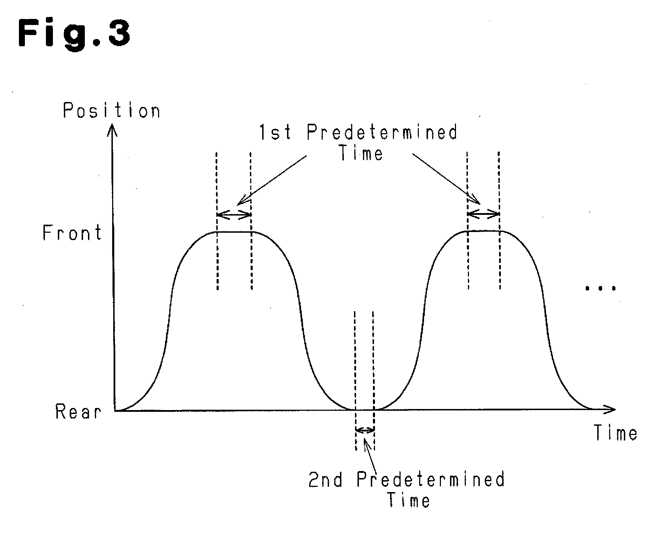

Fig. 3 is a time chart showing the position of the body support 13 during rocking. In the example of Fig. 3, the controller 24 controls the motor 20 so that the first predetermined time is longer than the second predetermined time. Through such controlling, the first predetermined time, during which a floating feel is generally easy to obtain, becomes longer than the second predetermined time. Thus, the user obtains a further floating feel.

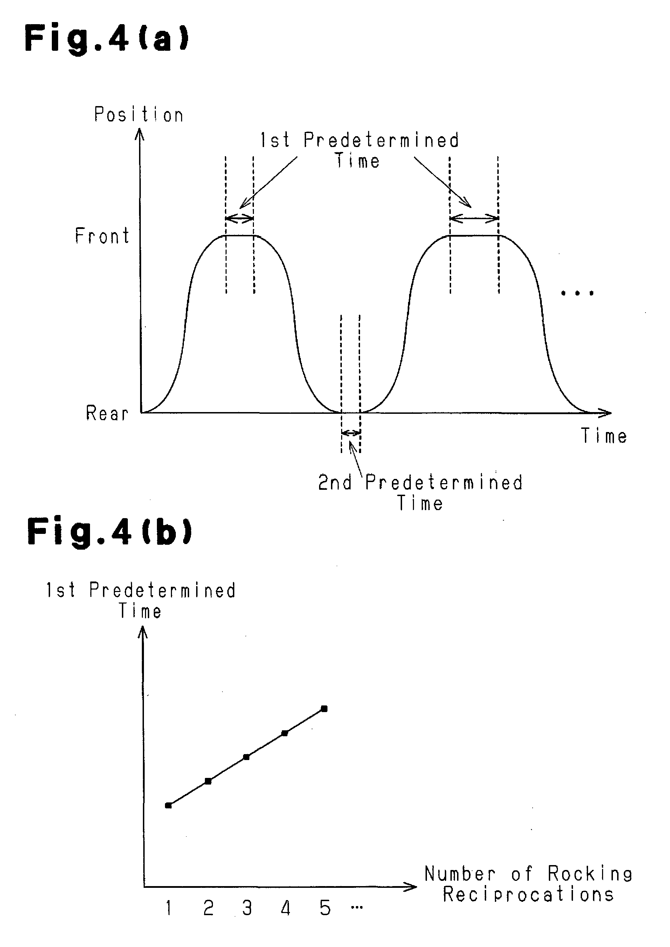

The controller 24 may control the motor 20 so that at least either one of the first predetermined time and the second predetermined time is gradually increased while repeating relaxation rocking. In the examples of Figs. 4(a) and 4(b), as the number of rocking reciprocations of the relaxation device 10 increases, the controller 24 increases the first predetermined time. In a further example, as the number of rocking reciprocations of the relaxation device 10 increases, the controller 24 increases only the second predetermined time and does not increase the first predetermined time. In a further example, as the number of rocking reciprocations increases, the controller 24 increases both of the first predetermined time and the second predetermined time.

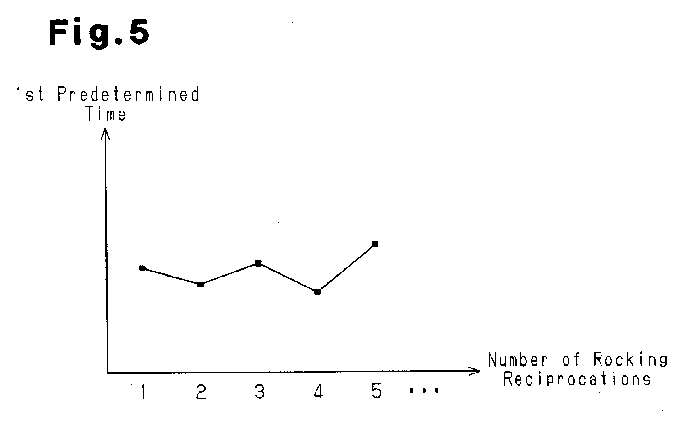

In the example of Fig. 5, the controller 24 may increase and decrease the first predetermined time in an irregular manner when rocking is repeated to vary the first predetermined time in a fluctuated manner. The controller 24 may vary the second predetermined time in a fluctuated manner. The controller 24 may vary both of the first predetermined time and the second predetermined time. Normally, the varying width of the rocking operation is 0.2 Hz or less but not limited in such a manner. Further, in addition to changing the first predetermined time and the second predetermined time, as the number of rocking reciprocation increases, the controller 24 may increase the rocking amplitude, the rocking frequency, or both of the rocking amplitude and rocking frequency.

The first embodiment has the advantages described below.
(1) When changing the rocking direction of the body support 13, the controller 24 controls the rocking driver 12 to stop the rocking of the body support 13 for a predetermined time. By temporarily stopping the rocking, the user can obtain a floating feel and enjoy the rocking. Further, the controller 24 controls the rocking driver 12 so that the first predetermined time for when the rocking direction of the body support 13 changes from the forward direction to the rearward direction differs from the second predetermined time for when the rocking direction of the body support 13 changes from the rearward direction to the forward direction. Thus, the user can obtain a variety of floating feels.
(2) The controller 24 controls the rocking driver 12 so that the first predetermined time is longer than the second predetermined time. Thus, the user becomes more aware of the stopped movement during the first predetermined time, and the user obtains a further floating feel.
(3) While repeating relaxation rocking, the controller 24 controls the rocking driver 12 to gradually increase at least either one of the first predetermined time and the second predetermined time. Thus, the user obtains a gradually increasing floating feel.

A second embodiment will now be described. The relaxation device 10 of the second embodiment has the same structure as the first embodiment and thus will not be described.

In the relaxation device of the second embodiment, the controller 24 controls the lifting mechanism and the rocking drive unit so that the user is lifted by the lifting mechanism during either one of the first predetermined time and the second predetermined time. For example, the controller 24 controls the air pump 49 so that the airbags 40 to 48 are inflated during at least either one of the first predetermined time and the second predetermine time.

For example, the airbags 40 to 48 are inflated and deflated under the control of the controller 24 in the following manner. The airbags 40 to 48 are inflated during the first predetermined time in which the rocking direction of the body support 13 changes from the front direction to the rear direction. As the body support 13 starts to rock toward the rear direction, the airbags 40 to 48 start to deflate. When the deflation ends, the airbags 40 to 48 start to inflate again. The airbags 40 to 48 are inflated during the second predetermined time. Then, when the body support 13 starts to rock in the forward direction, the airbags 40 to 48 are deflated again. When the deflation ends, the airbags 40 to 48 start to inflate again, and the airbags 40 to 48 are inflated during the first predetermined time. The relaxation device 10 includes a position sensor that detects the position of the body support 13 when rocked. For example, in accordance with a detection signal of the position sensor, the controller 24 controls the lifting mechanism and the rocking drive unit to end the deflation of the airbags 40 to 48 when the body support 13 is located at the lowermost point in the arc-shaped movement path.

Further, the controller 24 may control the air pump 49 (airbags 40 to 48) in the following manner. At a timing when the rocking direction of the body support 13 changes from the forward direction to the rearward direction, the controller 24 starts to inflate the airbags 40 to 48. As the body support 13 rocks in the rearward direction, the controller 24 inflates the airbags 40 to 48 so that the airbags 40 to 48 are in a desired inflation state (e.g., a substantially complete inflation state) just before the body support 13 reaches a rearmost point or when the body support 13 is stayed in the rearmost point (i.e., second predetermined time). Then, the airbags 40 to 48 are temporarily deflated just before the rearmost point or at the rearmost point. At a timing when the rocking direction of the body support 13 shifts from the rearward direction to the forward direction, the controller 24 starts to inflate the airbags 40 to 48. As the body support 13 rocks in the forward direction, the controller 24 inflates the airbags 40 to 48 so that the airbags 40 to 48 are in a desired inflation state (e.g., a substantially complete inflation state) just before the body support 13 reaches a frontmost point or when the body support 13 is stayed in the frontmost point (i.e., first predetermined time). Then, the airbags 40 to 48 are temporarily deflated just before the frontmost point or at the frontmost point.

The controller 24 may inflate the airbags 40 to 48 in both of the first predetermined time and the second predetermined time but may also inflate the airbags 40 to 48 in the first predetermined time and deflate the airbags 40 to 48 in the second predetermined time. Alternatively, the controller 24 may inflate the airbags 40 to 48 in the second predetermined time and deflate the airbags 40 to 48 in the first predetermined time. To inflate the airbags 40 to 48 during only the first predetermined time, the following control can be executed. The airbags 40 to 48 are deflated during the second predetermined time when the rocking direction of the body support 13 changes from the rearward direction to the forward direction. At a timing when the rocking direction of the body support 13 is reversed from the rearward direction to the forward direction, the controller 24 starts to inflate the airbags 40 to 48 so that the airbags 40 to 48 are in a desired inflation state at the first predetermined time. Then, at a timing at which the body support 13 starts to rock in the rearward direction, that is, at a timing at which the rocking direction is reversed, the controller 24 starts to control deflation of the airbags 40 to 48 and deflates the airbags 40 to 48 as the body support 13 rocks toward the rearward direction so that the airbags 40 to 48 are in a desired inflation state (e.g., substantially complete deflation state) during the second predetermined time.

In addition to the advantages of the second embodiment, the relaxation device of the second embodiment has the advantages described below.
(4) The controller 24 inflates the airbags 40 to 48 in cooperation with when the rocking driver 12 temporarily stops the rocking. When rocking is being performed, the user is lifted by the airbags 40 to 48 during at least either one of the first predetermined time and the second predetermined time. Thus, the user obtains a further floating feel.
(5) The airbags 40 to 48, which lift the user, are arranged in the body support 13. The controller 24 controls the inflation and deflation of the airbags 40 to 48 so that the user is lifted during at least either one of the first predetermined time and the second predetermined time. Thus, the user obtains a further floating feel.

A third embodiment will now be described. The relaxation device 10 of the present embodiment has the same structure as the first and second embodiments and thus will not be described. Operations of the relaxation device 10 similar to those of the first and second embodiments will not be described. In the relaxation device 10 of the third embodiment, the controller 24, which serves as a control unit, controls the reclining mechanism 34 and reclines the backrest 31 so that the backrest 31 is reclined during at least other one of the first predetermined time and the second predetermined time.

More specifically, in either one of the first predetermined time and the second predetermined time, the controller controls the reclining mechanism 34 and reclines the backrest 31 by a predetermined angle (e.g., 30 degrees) so that a reclining angle θ between the seat 30 and the backrest 31 changes from an upright state (e.g., reclining angle θ = 120 degrees) to a reclining state (e.g., reclining angle θ = 150 decrees).

The reclining mechanism 34 may be controlled to recline the backrest 31 during only the first predetermined time, during only the second predetermined time, or during only the first predetermined time and second predetermined time.

The relaxation device of the third embodiment has the advantages described below. (6) The controller 24 reclines the backrest 31 so that the backrest 31 of the body support 13 is in a reclining state during at least either one of the first predetermined time and the second predetermined time. The user obtains a further floating feel and a released feel during at least either one of the first predetermined time and the second predetermined time.

Program codes for executing the controls of each embodiment described above and the predetermined times may be stored in a memory of the controller 24.

### DESCRIPTION OF REFERENCE CHARACTERS

10: relaxation device
12: rocking driver (rocking drive unit)
13: body support (body support unit)
24: controller (control unit)
31: backrest
40 to 48: airbags (lifting mechanism)

## Claims

1. A relaxation device (10) including a body support unit (13) adapted to support a user's body and including a seat (30), a backrest (31), an ottoman (32) and armrests (33), the relaxation device (10) being **characterized by**:
a rocking drive unit (12) that rocks the body support unit (13) toward the front and rear along an arc-shaped movement path, and a control unit (24) that controls the rocking drive unit (12) to perform relaxation rocking with the body support unit (13),
wherein the control unit (24) controls the rocking drive unit (12) so that the rocking of the body support unit (13) is stopped for a predetermined time when a rocking direction of the body support unit (13) changes; and
the control unit (24) controls a first predetermined time, for when the rocking direction of the body support unit including the seat and the backrest changes from a forward direction to a rearward direction, to be different from a second predetermined time, for when the rocking direction of the body support unit including the seat and the backrest changes from the rearward direction to the forward direction.

2. The relaxation device (10) according to claim 1, being **characterized in that** the control unit (24) controls the rocking drive unit (12) so that the first predetermined time becomes longer than the second predetermined time.

3. The relaxation device (10) according to claim 1 or 2, being **characterized in that** the control unit (24) gradually increases at least either one of the first predetermined time and the second predetermined time as the relaxation rocking is repeated.

4. The relaxation device (10) according to claim 1 or 2, being **characterized in that** the control unit (24) increases and decreases at least either one of the first predetermined time and the second predetermined time in an irregular manner as the relaxation rocking is repeated.

5. The relaxation device (10) according to any one of claims 1 to 4, being further **characterized by** a lifting mechanism (40 - 48, 49) arranged in the body support unit (13) to lift the user, wherein the control unit (24) controls the lifting mechanism (40 - 48, 49) so that the user is lifted in at least either one of the first predetermined time and the second predetermined time.

6. The relaxation device (10) according to any one of claims 1 to 5, being **characterized in that** the body support unit includes the seat (30) on which a user can sit and the backrest (31) fixed in a reclinable manner to the rear of the seat (30), and the control unit (24) reclines the backrest (31) to set the backrest in a reclining state during at least either one of the first predetermined time and the second predetermined time.

## Patentansprüche

1. Entspannungsvorrichtung (10), die eine Körperauflageeinheit (13), die geeignet ist, um den Körper eines Benutzers abzustützen, und einen Sitz (30), eine Rückenlehne (31), einen Hocker (32) und Armlehnen (33) umfasst, wobei die Entspannungsvorrichtung (10) **gekennzeichnet ist durch**:
eine Schaukelantriebseinheit (12), welche die Körperauflageeinheit (13) entlang eines bogenförmigen Bewegungspfads hin und her schaukelt, und eine Steuereinheit (24), welche die Schaukelantriebseinheit (12) steuert, um ein entspannendes Schaukeln mit der Körperauflageeinheit (13) auszuführen,
wobei die Steuereinheit (24) die Schaukelantriebseinheit (12) derart steuert, dass das Schaukeln der Körperauflageeinheit (13) eine vorbestimmte Zeit lang angehalten wird, wenn sich eine Schaukelrichtung der Körperauflageeinheit (13) ändert; und
die Steuereinheit (24) einen ersten vorbestimmten Zeitraum, während dessen sich die Schaukelrichtung der Körperauflageeinheit, die den Sitz und die Rückenlehne umfasst, von einer Richtung nach vorne in eine Richtung nach hinten ändert, derart steuert, dass er anders als ein zweiter vorbestimmter Zeitraum ist, während dessen sich die Schaukelrichtung der Körperauflageeinheit, die den Sitz und die Rückenlehne umfasst, von der Richtung nach hinten in die Richtung nach vorne ändert.

2. Entspannungsvorrichtung (10) nach Anspruch 1, **dadurch gekennzeichnet, dass** die Steuereinheit (24) die Schaukelantriebseinheit (12) derart steuert, dass der erste vorbestimmte Zeitraum länger wird als der zweite vorbestimmte Zeitraum.

3. Entspannungsvorrichtung (10) nach Anspruch 1 oder 2, **dadurch gekennzeichnet, dass** die Steuereinheit (24) allmählich mindestens einen von dem ersten vorbestimmten Zeitraum und dem zweiten vorbestimmten Zeitraum erhöht, während das entspannende Schaukeln wiederholt wird.

4. Entspannungsvorrichtung (10) nach Anspruch 1 oder 2, **dadurch gekennzeichnet, dass** die Steuereinheit (24) mindestens einen von dem ersten vorbestimmten Zeitraum und dem zweiten vorbestimmten Zeitraum unregelmäßig erhöht und verringert, während das entspannende Schaukeln wiederholt wird.

5. Entspannungsvorrichtung (10) nach einem der Ansprüche 1 bis 4, ferner **gekennzeichnet durch** einen Hebemechanismus (40 - 48, 49), der in der Körperauflageeinheit (13) angeordnet ist, um den Benutzer anzuheben, wobei die Steuereinheit (24) den Hebemechanismus (40 - 48, 49) derart steuert, dass der Benutzer in mindestens einem von dem ersten vorbestimmten Zeitraum und dem zweiten vorbestimmten Zeitraum angehoben wird.

6. Entspannungsvorrichtung (10) nach einem der Ansprüche 1 bis 5, **dadurch gekennzeichnet, dass** die Körperauflageeinheit den Sitz (30), auf dem ein Benutzer sitzen kann, und die Rückenlehne (31), die zurücklehnbar hinten am Sitz (30) befestigt ist, umfasst, und die Steuereinheit (24) die Rückenlehne (31) zurücklehnt, um die Rückenlehne in einen zurückgelehnten Zustand während mindestens einem von dem ersten vorbestimmten Zeitraum und dem zweiten vorbestimmten Zeitraum einzustellen.

## Revendications

1. Dispositif de relaxation (10) comprenant une unité de support de corps (13) adaptée pour supporter le corps d'un utilisateur et comprenant un siège (30), un dossier (31), une ottomane (32) et des accoudoirs (33), le dispositif de relaxation (10) étant **caractérisé par** :
une unité d'entraînement de balancement (12) qui balance l'unité de support de corps (13) vers l'avant et l'arrière le long d'une trajectoire de mouvement en forme d'arc, et une unité de commande (24) qui commande l'unité d'entraînement de balancement (12) pour réaliser le balancement de relaxation avec l'unité de support de corps (13),
dans lequel l'unité de commande (24) commande l'unité d'entraînement de balancement (12) de sorte que le balancement de l'unité de support de corps (13) est arrêté pendant une période de temps prédéterminée lorsqu'une direction de balancement de l'unité de support de corps (13) change ; et
l'unité de commande (24) commande une première période de temps prédéterminée, pendant laquelle la direction de balancement de l'unité de support de corps comprenant le siège et le dossier passe d'une direction vers l'avant à une direction vers l'arrière qui est différente d'une seconde période de temps prédéterminée, pendant laquelle la direction de balancement de l'unité de support de corps comprenant le siège et le dossier passe de la direction vers l'arrière à la direction vers l'avant.

2. Dispositif de relaxation (10) selon la revendication 1, **caractérisé en ce que** l'unité de commande (24) commande l'unité d'entraînement de balancement (12) de sorte que la première période de temps prédéterminée devient plus longue que la seconde période de temps prédéterminée.

3. Dispositif de relaxation (10) selon la revendication 1 ou 2, **caractérisé en ce que** l'unité de commande (24) augmente progressivement au moins l'une parmi la première période de temps prédéterminée et la seconde période de temps prédéterminée lorsque le balancement de relaxation est répété.

4. Dispositif de relaxation (10) selon la revendication 1 ou 2, **caractérisé en ce que** l'unité de commande (24) augmente et diminue au moins chacune parmi la première période de temps prédéterminée et la seconde période de temps prédéterminée d'une manière irrégulière lorsque le balancement de relaxation est répété.

5. Dispositif de relaxation (10) selon l'une quelconque des revendications 1 à 4, **caractérisé en outre par** un mécanisme de levage (40-48, 49) agencé dans l'unité de support de corps (13) pour lever l'utilisateur, dans lequel l'unité de commande (24) commande le mécanisme de levage (40-48, 49) de sorte que l'utilisateur est levé dans au moins chacune parmi la première période de temps prédéterminée et la seconde période de temps prédéterminée.

6. Dispositif de relaxation (10) selon l'une quelconque des revendications 1 à 5, **caractérisé en ce que** l'unité de support de corps comprend le siège (30) sur lequel un utilisateur peut s'assoir et le dossier (31) fixé d'une manière inclinable à l'arrière du siège (30), et l'unité de commande (24) incline le dossier (31) pour régler le dossier dans un état incliné pendant au moins chacune parmi une première période de temps prédéterminée et la seconde période de temps prédéterminée.
